# EUROPÄISCHE PATENTSCHRIFT

(11) **EP 0 816 312 B1**
(45) Veröffentlichungstag und Bekanntmachung des Hinweises auf die Patenterteilung: **31.10.2001**
(21) Anmeldenummer: 97109997.3
(22) Anmeldetag: 19.06.1997
(51) Int. Cl.: C07C 17/16, C07C 17/38, C07C 17/386, C07C 17/383, C07C 19/03, C07C 41/09, C07C 41/42, C07C 43/04

(54) **Verfahren zur Herstellung und Auftrennung eines Gemischs aus Dimethylether und Chlormethan mit Wasser als Extraktionsmittel**
Process for the preparation and separation of a mixture of dimethyl ether and chloromethan using water as the extractant
Procédé pour la préparation et la séparation d'un mélange d'éther diméthylique et de chlorométhane au moyen d'eau comme agent d'extraction

(30) Priorität: 25.06.1996 DE 19625283
(43) Veröffentlichungstag der Anmeldung: 07.01.1998
(73) Patentinhaber: HOECHST AKTIENGESELLSCHAFT, 65929 Frankfurt am Main (DE)
(72) Erfinder: Roth, Peter, Dl., 65817 Eppstein (DE); Leistner, Erhard, Dl., 35619 Braunfels (DE); Haverkamp, Hans, Dl., 65817 Eppstein (DE)
(74) Vertreter: Schweitzer, Klaus, Dr.

(56) Entgegenhaltungen:
- EP-A- 0 039 001
- EP-A- 0 124 078
- US-A- 2 421 441
- US-A- 4 220 609
- US-A- 5 092 966
- DATABASE WPI Section Ch, Week 8036 Derwent Publications Ltd., London, GB; Class E16, AN 80-62426C XP002040756 & DD 142 183 A (BERGER K H) , 11.Juni 1980

## Beschreibung

Die vorliegende Erfindung betrifft ein Verfahren zur Herstellung eines Gemischs aus Dimethylether und Chlormethan, sowie dessen Auftrennung mittels Extraktivdestillation mit Wasser als Extraktionsmittel.

Chlormethan besitzt technische Bedeutung als Ausgangsprodukt für die Herstellung von Fluorchlorkohlenwasserstoffen, die dann als Treibgase in Aerosolpackungen Verwendung finden. Dimethylether wird im zunehmendem Maße als Treibmittel in Aerosolpackungen eingesetzt, da es halogenfrei ist, und somit ein geringes Ozonabbaupotential aufweist.

Beide Verbindungen entstehen gemäß den Formelzeilen (I) und (II) bei der Umsetzung von Methanol mit HCI, die technisch zur Reaktionsbeschleunigung häufig an γ-Al₂O₃-Kontakten durchgeführt wird:

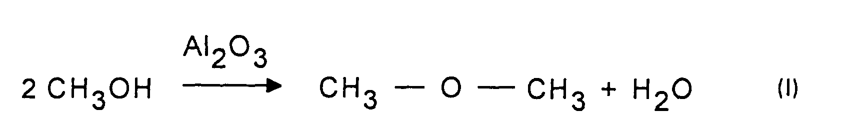

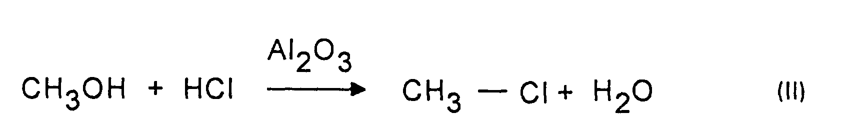

Bislang war es nach dem Stand der Technik üblich, den bei der Chlormethanherstellung anfallenden Dimethylether als Abfall anzusehen, und ihn durch Hydrolyse mit Schwefelsäure zu beseitigen. Da Dimethylether wegen seiner Verwendbarkeit als Treibmittel aber wertvoll ist; bestand der Bedarf für ein Verfahren, das den Zwangsanfall an Dimethylether technisch nutzbar macht.

Die Mischung aus Dimethylether und Chlormethan ist nach dem Stand der Technik destillativ nicht aufarbeitbar, da die Siedepunkte der Komponenten sehr nahe beieinander liegen (Dimethyether Kp= - 24,9 °C, Chlormethan Kp= - 23,7 °C), und die Komponenten außerdem ein Azetrop bilden.

DD-142 183 beschreibt ein Verfahren zur Trennung von Wasser, Methanol und den Chlormethanen Methylenchlorid, Chloroform und Tetrachlormethan durch Extraktivdestillation.

Die Aufgabe bestand somit darin, ein Herstellungs- und Trennverfahren zu entwickeln, das sowohl reinen Dimethylether als auch reines Chlormethan ergibt.

Diese Aufgabe wurde erfindungsgemäß durch ein zweistufiges Syntheseverfahren mit angeschlossenem dreistufigen Aufarbeitungsverfahren gelöst, das eine Extraktivdestillation mit Wasser beinhaltet.

Gegenstand der Erfindung ist somit ein Verfahren zur Herstellung von Dimethylether und Chlormethan durch Umsetzung von Methanol mit HCl und Abstrennung des entstehenden gemisches, dadurch gekennzeichnet, daß man
a) Methanol mit einem Überschuß an HCI umsetzt,
b) das in Schritt a) erhaltene, Wasser enthaltende Gemisch mit einem Überschuß an Methanol umsetzt,
c) das in Schritt b) erhaltene Gemisch einer Extraktivdestillationskolonne zuführt,
d) im oberen Teil der Extraktivdestillationskolonne Wasser als Extraktionsmittel aufgibt,
e) vom Kopf der Extraktivdestillationskolonne Chlormethan abzieht,
f) vom Sumpf der Extraktivdestillationskolonne ein Gemisch aus Wasser, Methanol und Dimethylether abzieht und einer ersten Destillationskolonne zuführt,
g) vom Kopf der ersten Destillationskolonne Dimethylether abzieht,
h) vom Sumpf der ersten Destillationskolonne ein Gemisch aus Wasser und Methanol abzieht,
i) das in Schritt h) erhaltene Gemisch in eine zweite Destillationskolonne einspeist,
k) am Kopf der zweiten Destillationskolonne reines Methanol entnimmt,
l) am Sumpf der zweiten Destillationskolonne Wasser entnimmt,
m) das in Schritt k) erhaltene Methanol den in den Schritten a) und b) erwähnten Reaktionen zuführt.

Die Verwendung eines Katalysators für die in den Schritten a) und b) beschriebene Veresterungsreaktion ist bevorzugt. Es kommen beispielsweise Y-Al₂O₃-Kontakte in Frage.

Bevorzugt geschieht die in Schritt c) beschriebene Gemischförderung dadurch, daß man gasförmig anfallendes Gemisch in einem Kondensator verflüssigt, das Kondensat mit einer Pumpe fördert, und es vor dem Eintritt in die Extraktivdestillationskolonne wieder verdampft.

Vorzugsweise wird die Extraktivdestillation bei Drucken zwischen 1 und 25 bar durchgeführt. Alle verwendeten Kolonnen können von einer beliebigen geeigneten Bauart sein, bevorzugt ist die Verwendung von Füllkörper- oder Packungskolonnen.

In einer Ausführungsform der Erfindung wird die Extraktivdestillationskolonne so betrieben, daß in Schritt g) Dimethylether und Methanol über Kopf abgezogen werden können. Der so erhaltene Produktstrom wird teilweise entnommen und der in den Schritten a) und b) beschriebenen Reaktion zugeführt. Diese Ausführungsform erlaubt mittelts einer Gleichgewichtsverschiebung eine Beeinflussung des Verhältnisses der produzierten Mengen an Chlormethan und Dimethylether.

In Figur I ist ein Fließschema für eine bevorzugte Ausführungsform des erfindungsgemäßen Verfahrens dargestellt, das im folgenden erläutert wird.

Einem zweistufigen Veresterungsreaktor (6) wird dampfförmiges Methanol (1) über Leitung (3) zugeführt. Außerdem wird gasförmiges HCI (2) über Leitung (5) in den Reaktor (6) eingespeist. Der zweistufige Veresterungsreaktor kann in Form eines zweistufigen Rohrreaktors ausgeführt sein. In der ersten Stufe werden HCI und Methanol in den Reaktor aufgegeben. Die Mengen der Reaktanden sind so bemessen, daß die Stoffmenge an HCI bis zu 20% über der stöchiometrisch zur Reaktion mit dem Methanol nötigen Stoffmenge liegt. Das Gemisch aus HCI und Methanol durchströmt die erste Stufe des Reaktors, die mit einem Katalysator beaufschlagt sein kann. Im Fall einer Gasphasenreaktion verläuft die Reaktion exotherm, so daß eine Kühlung für beide Stufen des Reaktors vorzusehen ist. Das die erste Stufe des Reaktors verlassende Gemisch aus Chlormethan, Wasser und HCI wird mit einer Menge an Methanol gemischt, die bis zu 20% über der Stoffmenge liegt, die stöchiometrisch zur Reaktion mit dem im Gemisch enthaltenen HCI nötig ist. Das Gemisch durchströmt die zweite Stufe des Reaktors, die ebenfalls mit einem Katalysator beaufschlagt sein kann. Die Reaktion kann mit flüssigen oder gasförmigen Reaktanden durchgeführt werden. Im Reaktor (6) laufen die in den Formelzeilen (I) und (II) angegebenen Reaktionen ab. Das den Reaktor verlassende, aus Dimethylether, Chlormethan, Methanol und Wasser bestehende Gemisch wird dann in die Extraktivdestillationskolonne (17) geleitet. Vorzugsweise wird das Gemisch über Leitung (10) zu einem Kondensator (11) geführt und dort verflüssigt, wo es über Leitung (12), eine Pumpe (13) und Leitung (14) zu einem Verdampfer (15) gelangt, bevor es über Leitung (16) in die Extraktivdestillationskolonne (17) eingespeist wird. In der Extraktivdestillationskolonne (17), deren Sumpf mittels des Verdampfers (18) beheizt wird, erfolgt eine Trennung von Dimethylether und Chlormethan. Über Leitung (26) wird im oberen Teil der Extraktivdestillationskolonne (17) Wasser als Extraktionsmittel aufgegeben, dessen Temperatur vorzugsweise zwischen 5 und 50 °C liegt. Wasser schlägt Dimethylether im Sumpf nieder, während Chlormethan als Kopfdampf über Leitung (20) abgezogen und gegebenenfalls dem Kondensator (21) zugeführt wird. Falls der Kopfdampf große Mengen Wasser enthält, was von den Betriebsbedingungen der Extraktivdestillationskolonne (17) abhängt, kann das Kondensat über Leitung (22) in einen Phasenabscheider (23) geleitet werden. Wird ein Phasenabscheider verwendet, so führt man die wäßrige Phase über Leitung (24) auf den Kopf der Extraktivdestillationskolonne (17) zurück. Chlormethan wird dann über Leitung (25) dem Produktlager (43) zugeführt. Wird kein Phasenabscheider verwendet, so kann man einen Teil des Kondensats über Leitung (24) als Rücklauf auf den Kopf der Extraktionsdestillationskolonne (17) zurückführen, entnimmt den verbleibenden Teil, und führt diesen über Leitung (25) dem Produktlager (43) zu. Im Sumpf der Extraktivdestillationskolonne (17) sammelt sich ein Gemisch aus Methanol, Dimethylether und Wasser. Dieses wird über Leitung (19), Pumpe (30) und Leitung (31) gegebenenfalls einem Wärmetauscher (29) zugeführt, in dem es erhitzt wird, und gelangt von dort über Leitung (32) zur ersten Destillationskolonne (33), in der Dimethylether abgetrennt wird. Dimethylether wird als Kopfdampf über Leitung (38) abgenommen und gegebenenfalls einem Kondensator (39) zugeführt. Von dort kann es zum Teil über die Leitungen (40) und (41) als Rücklauf wieder auf den Kopf der ersten Destillationskolonne (33) gelangen, sonst wird es über Leitung (42) entnommen und dem Produktlager (44) zugeführt. Im Sumpf der ersten Destillationskolonne (33), der über den Verdampfer (34) beheizt wird, sammelt sich ein Gemisch aus Methanol und Wasser. Dieses Gemisch wird über Leitung (35) einer Pumpe (36) zugeführt, und geht von dort über Leitung (46) gegebenenfalls zum Teil über Leitung (37) in den Wärmetauscher (29), wo es abgekühlt wird, und gegebenenfalls von dort über Leitung (28) in den Kühler (27), wo eine weitere Abkühlung stattfindet. Von dort wird es über Leitung (26) erneut in die Extraktivdestillationskolonne (17) als Extraktionsmittel aufgegeben. Zum Anfahren der Anlage und zur Ergänzung von Verlusten wird frisches Wasser (58) über Leitung (57) zugeführt. Der verbleibende Teil des Sumpfproduktes der ersten Destillationskolonne (33) wird über Leitung (47) einer zweiten Destillationskolonne (48) zugeführt, in der eine Trennung von Methanol und Wasser erfolgt. Die zweite Destillationskolonne wird vorzugsweise drucklos betrieben. Als Kopfdampf der zweiten Destillationskolonne (48) entnimmt man über Leitung (51) reines Methanol. Dieses kann im Kondensator (52) verflüssigt und zum Teil über Leitung (53) auf den Kopf der zweiten Destillationskolonne (48) zurück geleitet werden. Der verbleibende Teil des Methanols wird über Leitung (54) dem Veresterungsreaktor (6) zugeführt. Im Sumpf der zweiten Destillationskolonne (48) sammelt sich reines Wasser (55) an. Diese wird über Leitung (50) abgeführt. Der Sumpf der zweiten Destillationskolonne (48) wird über den Verdampfer (49) beheizt.

In einer besonderen Ausführungsform der Erfindung kann ausschließlich Chlormethan hergestellt werden. Dazu wird die erste Destillationskolonne (33) in der Weise betrieben, daß Dimethylether und Methanol über Kopf abdestilliert werden. Das entstandene Gemisch wird über Leitung (45) dem Veresterungsreaktor (6) zugeführt, wo durch eine Verschiebung des Reaktionsgleichgewichts dann hauptsächlich Chlormethan entsteht. Ein Teil des aus der ersten Destillationskolonne austretenden Reaktionswassers wird dann über die Leitungen (47), (56) und (50) direkt entnommen. In dieser speziellen Ausführungsform der Erfindung ist die zweite Destillationskolonne (48) nicht erforderlich.

Die folgende Tabelle gibt eine Übersicht über die in den Kolonnen auftretenden Kopf- und Sumpftemperaturen in °C im bevorzugten Druckbereich.

| | 1 bar | | 25 bar | |
|---|---|---|---|---|
| Kolonne | Kopf | Sumpf | Kopf | Sumpf |
| (17) | -24 | 90 | 90 | 200 |
| (33) | -25 | 100 | 85 | 224 |
| (48) | 64 | 100 | 180 | 224 |

### Beispiel:

Dem Veresterungsreaktor (6) werden 7,1 t/h Methanol und 5,3 t/h HCI zugeführt. Die zweistufige Reaktion bei 250°C und einem Druck von 4 bar unter Verwendung eines γ-Al₂O₃-Katalysators bewirkt die Entstehung eines Reaktionsgases folgender Zusammensetzung:

| | |
|---|---|
| Dimethylether | 1,8 t/h |
| Methanol | 0,01 t/h |
| Chlormethan | 7,3 t/h |
| H₂O | 3,29 t/h |

Dieses Reaktionsgas wird bei etwa 4 bar und einer tiefsten Temperatur von etwa 10°C vollständig im Kondensator (11) verflüssigt. Das anfallende Kondensat wird mit der Pumpe (13) in den Verdampfer (15) gefördert. Hier erfolgt die teilweise Verdampfung des Kondensats bei 10 bar und etwa 100°C. Als Heizmedium kann hierfür die im Kondensator (11) freiwerdende Kondensationswärme verwendet werden. Das Dampf/Flüssigkeitsgemisch aus (15) wird vollständig in die Extraktivdestillationskolonne (17) eingespeist. Diese wird ebenfalls bei einem Druck von 10 bar betrieben.

Sollte aus unvorhergesehenen Gründen HCI im Reaktionsgas auftreten, kann dieses im Kondensator (11) mittels wäßriger Natronlauge neutralisiert werden. In der Extraktivdestillationskolonne (17) wird Dimethylether in etwa 36 t/h Extraktionswasser mit einem Methanol-Anteil von etwa 2,4 Gew.-% gelöst. Da sich gleichzeitig im Extraktionsmittel eine gewisse Menge Chlormethan löst, wird dieses im unteren Teil der Kolonne ausgestrippt. Die Beheizung erfolgt mittels Heizdampf über den Verdampfer (18). Als Sumpfprodukt der Extraktivdestillation fällt ein Gemisch, bestehend aus:

| | |
|---|---|
| 1,8 t/h | Dimethylether |
| 0,9 t/h | Methanol |
| 39,0 t/h | Wasser |

an. Der Anteil an Chlormethan in diesem Produktstrom liegt bei etwa 1 ppm.

Als flüssiges Kopfprodukt der Extraktivdestillationskolonne (17) fallen 7,3 t/h Chlormethan mit einem Dimethylether-Anteil von weniger als 20 ppm an. Das Extraktionswasser muß möglichst kalt über Leitung (26) eingespeist werden, vorzugsweise mit etwa 35°C. Die Kopftemperatur der Extraktivdestillationskolonne (17) beträgt etwa 40°C, was die Kondensation des Kopfproduktes mittels Rückkühlwasser ermöglicht. Die Sumpftemperatur beträgt etwa 124°C, so daß die Beheizung mit Niederdruck-Dampf möglich ist.

Mit Hilfe der Pumpe (30) wird der Sumpfablauf aus der Extraktivdestillationskolonne (17) über den Wärmetauscher (29) vorgewärmt und in die Kolonne (33) eingespeist. Diese wird ebenfalls bei 10 bar betrieben. Als Kopfprodukt fallen hier etwa 1,8 t/h flüssiger Dimethylether bei etwa 40°C an. Der Anteil Chlormethan im Dimethylether liegt bei weniger als 20 ppm. Das Sumpfprodukt aus der ersten Destillationskolonne (33) ist ein Gemisch bestehend aus etwa 40 t/h Wasser mit einem Methanolanteil von etwa 2,4 Gew.-%. Es wird zum überwiegenden Teil (etwa 36 t/h) nach Kühlung auf etwa 35°C erneut in die Extraktivdestillationskolonne (17) eingespeist. Ein Teilstrom des Sumpfproduktes der ersten Destillationskolonne (33) wird in die zweite Destillationskolonne (48) eingeführt. Hier erfolgt bei einem Druck von 1 bar die Trennung des Methanols vom Wasser. Methanol (etwa 0,01 t/h) wird hier als Kopfprodukt gewonnen und erneut in die Reaktion (6) eingespeist. Das Sumpfprodukt ist Wasser (etwa 3,9 t/h) mit einem Methanol-Anteil von etwa 0,08 Gew.-%.

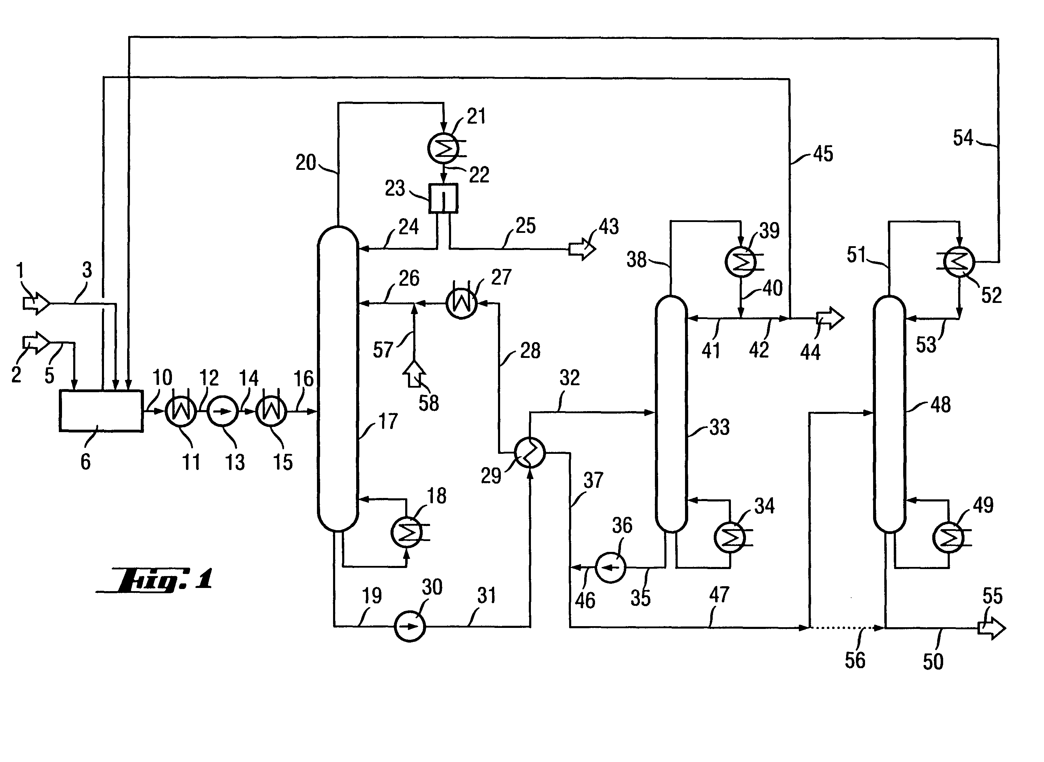

## Patentansprüche

1. Verfahren zur Herstellung von Dimethylether und Chlormethan durch Umsetzung von Methanol mit HCI und Abtrennung des entstehenden Gemisches, **dadurch gekennzeichnet, daß** man
a) Methanol mit einem Überschuß an HCI umsetzt,
b) das in Schritt a) erhaltene, Wasser enthaltende Gemisch mit einem Überschuß an Methanol umsetzt,
c) das in Schritt b) erhaltene Gemisch einer Extraktivdestillationskolonne zuführt,
d) im oberen Teil der Extraktivdestillationskolonne Wasser als Extraktionsmittel aufgibt,
e) vom Kopf der Extraktivdestillationskolonne Chlormethan abzieht,
f) vom Sumpf der Extraktivdestillationskolonne ein Gemisch aus Wasser, Methanol und Dimethylether abzieht und einer ersten Destillationskolonne zuführt,
g) vom Kopf der ersten Destillationskolonne Dimethylether abzieht,
h) vom Sumpf der ersten Destillationskolonne ein Gemisch aus Wasser und Methanol abzieht,
i) das in Schritt h) erhaltene Gemisch in eine zweite Destillationskolonne einspeist,
k) am Kopf der zweiten Destillationskolonne reines Methanol entnimmt,
l) am Sumpf der zweiten Destillationskolonne Wasser entnimmt,
m) das in Schritt k) erhaltene Methanol den in den Schritten a) und b) erwähnten Reaktionen zuführt.

2. Verfahren nach Anspruch 1, **dadurch gekennzeichnet, daß** die in den Schritten e) und g) erhaltenen Produkte vor ihrer weiteren Verwendung kondensiert werden.

3. Verfahren nach Anspruch 1 oder 2, **dadurch gekennzeichnet, daß** der in Schritt e) aus dem Kopf der Extraktionsdestillationskolonne austretende Produktstrom eine Phasentrennung durchläuft.

4. Verfahren nach einem der Ansprüche 1 bis 3, **dadurch gekennzeichnet, daß** während der in Schritt c) beschriebenen Zuführung gasförmig anfallendes Reaktionsgemisch zuerst kondensiert und dann wieder verdampft wird.

5. Verfahren zur Herstellung von Chlormethan, **dadurch gekennzeichnet, daß** man
a) Methanol mit einem Überschuß an HCI umsetzt,
b) das in Schritt a) erhaltene wasserhaltige Gemisch mit einem Überschuß an Methanol umsetzt,
c) das in Schritt b) erhaltene Gemisch einer Extraktionsdestillationskolonne zuführt,
d) im oberen Teil der Extraktionsdestillationskolonne Wasser als Extraktionsmittel aufgibt,
e) vom Kopf der Extraktionsdestillationskolonne Chlormethan abzieht,
f) vom Sumpf der Extraktionsdestillationskolonne ein Gemisch aus Wasser, Methanol und Dimethylether abzieht und einer Destillationskolonne zuführt,
g) vom Kopf der Destillationskolonne Dimethylether und Methanol abzieht,
h) vom Sumpf der ersten Destillationskolonne einen Teil des Wassers abzieht,
i) das in Schritt g) erhaltene Gemisch aus Dimethylether und Methanol den in den Schritten a) und b) erwähnten Reaktionen zuführt.

6. Verfahren nach Anspruch 5, **dadurch gekennzeichnet, daß** man das in Schritt g) erhaltene Gemisch zum Teil auf den Kopf der Destillationskolonne zurückführt.

7. Verfahren nach einem der Ansprüche 1 bis 6, **dadurch gekennzeichnet, daß** die Extraktionsdestillation nach Schritt c) bei Drucken zwischen 1 und 25 bar durchgeführt wird.

8. Verfahren nach einem der Ansprüche 1 bis 7, **dadurch gekennzeichnet, daß** alle verwendeten Kolonnen als Füllkörper- oder Packungskolonnen ausgeführt werden.

9. Verfahren nach einem der Ansprüche 1 bis 8, **dadurch gekennzeichnet, daß** das Extraktionswasser in Schritt d) mit einer Temperatur zwischen 5 und 50 °C in die Extraktivdestillation eingespeist wird.

10. Verfahren nach einem der Ansprüche 1 bis 9, **dadurch gekennzeichnet, daß** man das in Schritt f) beschriebene Gemisch vor der Aufgabe in die erste Destillationskolonne erwärmt, und daß man das in Scnritt h) erwähnte Gemisch vor der Aufgabe in die Extraktivdestillationskolonne abkühlt.

11. Verfahren nach Anspruch 10, **dadurch gekennzeichnet, daß** die Erwärmung des in Schritt f) beschriebenen Gemischs in einem Wärmetauscher erfolgt, und daß die Abkühlung des in Schritt h) beschriebenen Gemischs ebenfalls in dem Wärmetauscher erfolgt, und daß das in Schritt h) beschriebene Gemisch vor der Einspeisung in die Extraktivdestillationskolonne in einem Kühler abgekühlt wird.

12. Verfahren nach einem der Ansprüche 1 bis 11, **dadurch gekennzeichnet, daß** die in den Schritten a) und b) beschriebene Reaktion unter Zuhilfenahme eines Katalysators durchgeführt wird.

13. Verfahren nach einem der Ansprüche 1 bis 12, **dadurch gekennzeichnet, daß** die in den Schritten a) und b) genannten Eduktüberschüsse bis zu 20 % der zur Reaktion erforderlichen stöchiometrischen Mengen betragen.

14. Verfahren nach einem der Ansprüche 1 bis 13, **dadurch gekennzeichnet, daß** man
1) einen Teil des in Schritt e) erhaltenen Produkts auf den Kopf der Extraktivdestillationskolonne zurückführt,
und/oder
2) einen Teil des in Schritt g) erhaltenen Produkts auf den Kopf der ersten Destillationskolonne zurückführt,
und/oder
3) einen Teil des in Schritt h) erhaltenen Gemischs bzw. Wassers der Extraktivdestillationskolonne als Extraktivmittel zuführt,
und/oder
4) das in Schritt k) erhaltene Produkt teilweise dem Kopf der zweiten Destillationskolonne zuführt.

## Claims

1. A process for the preparation of dimethyl ether and chloromethane by reacting methanol with HCl and separating off the resulting mixture, which comprises
a) reacting methanol with an excess of HCl,
b) reacting the mixture containing water and obtained in step a) with an excess of methanol,
c) feeding the mixture obtained in step b) to an extractive distillation column,
d) adding water as extractant in the upper part of the extractive distillation column,
e) taking off chloromethane from the top of the extractive distillation column,
f) taking off a mixture of water, methanol and dimethyl ether from the bottom of the extractive distillation column and feeding it to a first distillation column,
g) taking off dimethyl ether from the top of the first distillation column,
h) taking off a mixture of water and methanol from the bottom of the first distillation column,
i) feeding the mixture obtained in step h) into a second distillation column,
k) taking off pure methanol at the top of the second distillation column,
l) taking off water at the bottom of the second distillation column,
m) feeding the methanol obtained in step k) into the reactions mentioned in steps a) and b).

2. The process as claimed in claim 1, wherein the products obtained in steps e) and g) are condensed before being used further.

3. The process as claimed in claim 1 or 2, wherein the product stream emerging from the top of the extractive distillation column in step e) undergoes a phase separation.

4. The process as claimed in any of claims 1 to 3, wherein the reaction mixture produced as gas during the feeding described in step c) is first condensed and then vaporized again.

5. A process for the preparation of chloromethane,which comprises
a) reacting methanol with an excess of HCl,
b) reacting the mixture containing water and obtained in step a) with an excess of methanol,
c) feeding the mixture obtained in step b) to an extractive distillation column,
d) adding water as extractant in the upper part of the extractive distillation column,
e) taking off chloromethane from the top of the extractive distillation column,
f) taking off a mixture of water, methanol and dimethyl ether from the bottom of the extractive distillation column and feeding it to a distillation column,
g) taking off dimethyl ether and methanol from the top of the distillation column,
h) taking off part of the water from the bottom of the first distillation column,
i) feeding the mixture of dimethyl ether and methanol obtained in step g) into the reactions mentioned in steps a) and b).

6. The process as claimed in claim 5, wherein the mixture obtained in step g) is partly returned to the top of the distillation column.

7. The process as claimed in any of claims 1 to 6, wherein the extractive distillation after step c) is carried out under pressures between 1 and 25 bar.

8. The process as claimed in any of claims 1 to 7, wherein all the columns used are designed as packed columns.

9. The process as claimed in any of claims 1 to 8, wherein the extraction water is at a temperature between 5 and 50°C when fed into the extractive distillation in step d).

10. The process as claimed in any of claims 1 to 9, wherein the mixture described in step f) is heated before addition to the first distillation column, and wherein the mixture mentioned in step h) is cooled before addition to the extractive distillation column.

11. The process as claimed in claim 10, wherein the heating of the mixture described in step f) takes place in a heat exchanger, and wherein the cooling of the mixture described in step h) likewise takes place in the heat exchanger, and wherein the mixture described in step h) is cooled in a cooler before being fed into the extractive distillation column.

12. The process as claimed in any of claims 1 to 11, wherein the reaction described in steps a) and b) is carried out with the aid of a catalyst.

13. The process as claimed in any of claims 1 to 12, wherein the excesses of precursors mentioned in steps a) and b) are up to 20% of the stoichiometric amounts required for the reaction.

14. The process as claimed in any of claims 1 to 13, wherein
1) part of the product obtained in step e) is returned to the top of the extractive distillation column,
and/or
2) part of the product obtained in step g) is returned to the top of the first distillation column,
and/or
3) part of the mixture or water obtained in step h) is fed as extractant to the extractive distillation column,
and/or
4) the product obtained in step k) is partly fed to the top of the second distillation column.

## Revendications

1. Procédé pour la préparation d'éther diméthylique et de chlorométhane par mise en réaction du méthanol avec HCl et fractionnement du mélange résultant, **caractérisé en ce que**
a) on fait réagir du méthanol avec un excès de HCl,
b) on fait réagir avec un excès de méthanol le mélange contenant de l'eau, obtenu dans l'étape a),
c) on envoie à une colonne de distillation extractive le mélange obtenu dans l'étape b),
d) on introduit de l'eau comme agent d'extraction dans la partie supérieure de la colonne de distillation extractive,
e) on évacue le chlorométhane de la tête de la colonne de distillation extractive,
f) on soutire du pied de la colonne de distillation extractive un mélange d'eau, méthanol et éther diméthylique, et on l'envoie à une première colonne de distillation,
g) on évacue l'éther diméthylique de la tête de la première colonne de distillation,
h) on soutire du pied de la première colonne de distillation un mélange d'eau et de méthanol,
i) on introduit dans une seconde colonne de distillation le mélange obtenu dans l'étape h),
k) on prélève du méthanol pur à la tête de la seconde colonne de distillation,
l) on soutire de l'eau au bas de la seconde colonne de distillation,
m) on envoie le méthanol obtenu dans l'étape k) aux réactions mentionnées dans les étapes a) et b).

2. Procédé selon la revendication 1, **caractérisé en ce que** les produits obtenus dans les étapes e) et g) sont condensés avant leur nouvelle utilisation.

3. Procédé selon la revendication 1 ou 2, **caractérisé en ce que** le courant de produit sortant de la colonne de distillation extractive dans l'étape e) traverse une phase de séparation.

4. Procédé selon l'une des revendications 1 à 3, **caractérisé en ce que** le mélange réactionnel apparaissant à l'état gazeux pendant l'envoi décrit dans l'étape c) est d'abord condensé et ensuite vaporisé de nouveau.

5. Procédé pour la production de chlorométhane, **caractérisé en ce que**
a) on fait réagir du méthanol avec un excès de HCl,
b) on fait réagir avec un excès de méthanol le mélange contenant de l'eau, obtenu dans l'étape a),
c) on envoie à une colonne de distillation extractive le mélange obtenu dans l'étape b),
d) on introduit de l'eau comme agent d'extraction dans la partie supérieure de la colonne de distillation extractive,
e) on évacue le chlorométhane de la tête de la colonne de distillation extractive,
f) on soutire du pied de la colonne de distillation extractive un mélange d'eau, méthanol et éther diméthylique, et on l'envoie à une première colonne de distillation,
g) on évacue l'éther diméthylique et le méthanol de la tète de la première colonne de distillation,
h) on soutire une partie de l'eau du pied de la première colonne de distillation,
i) on envoie le mélange d'éther diméthylique et de méthanol, obtenu dans l'étape g) aux réactions mentionnées dans les étapes a) et b).

6. Procédé selon la revendication 5, **caractérisé en ce qu'**on renvoie en partie le mélange obtenu dans l'étape g) à la tête de la colonne de distillation.

7. Procédé selon l'une des revendications 1 à 6, **caractérisé en ce que** la distillation extractive ayant lieu après l'étape c) est effectuée sous des pressions comprises entre 1 et 25 bars.

8. Procédé selon l'une des revendications 1 à 7, **caractérisé en ce** toutes les colonnes utilisées ont la configuration de colonnes à garnissage ou corps de remplissage.

9. Procédé selon l'une des revendications 1 à 8, **caractérisé en ce que** l'eau pour extraction dans l'étape d) est introduite à une température comprise entre 5 et 50°C dans la colonne de distillation extractive.

10. Procédé selon l'une des revendications 1 à 9, **caractérisé en ce qu'**on chauffe le mélange décrit dans l'étape f), avant l'introduction dans la première colonne de distillation, et **en ce qu'**on refroidit le mélange mentionné dans l'étape h), avant l'introduction dans la colonne de distillation extractive.

11. Procédé selon la revendication 10, **caractérisé en ce que** le chauffage du mélange décrit dans l'étape f) s'effectue dans un échangeur thermique, et **en ce que** le refroidissement du mélange décrit dans l'étape h) s'effectue également dans l'échangeur thermique, et **en ce que** le mélange décrit dans l'étape h) est refroidi dans un refroidisseur avant l'introduction dans la colonne de distillation extractive.

12. Procédé selon l'une des revendications 1 à 11, **caractérisé en ce que** la réaction décrite dans les étapes a) et b) est effectuée à l'aide d'un catalyseur.

13. Procédé selon l'une des revendications 1 à 12, **caractérisé en ce que** les excès de produit de départ mentionnés dans les étapes a) et b) vont jusqu'à 20 % des quantités stoechiométriques requises pour la réaction.

14. Procédé selon l'une des revendications 1 à 13, **caractérisé en ce que**
1) une partie du produit obtenu dans l'étape e) est renvoyée à la tête de la colonne de distillation extractive
et/ou
2) une partie du produit obtenu dans l'étape g) est renvoyée à la tête de la première colonne de distillation
et/ou
3) une partie de l'eau ou du mélange obtenus dans l'étape h) est envoyée, en tant qu'agent d'extraction, à la colonne de distillation extractive
et/ou
4) le produit obtenu dans l'étape k) est en partie envoyé à la tète de la seconde colonne de distillation.
